# EUROPEAN PATENT SPECIFICATION

(11) **EP 4 188 901 B1**
(45) Date of publication and mention of the grant of the patent: **25.12.2024**
(21) Application number: 21755395.7
(22) Date of filing: 28.07.2021
(51) Int. Cl.: C07C 51/43, C07C 53/12, C07C 53/08, B27K 3/36, C07C 51/44, B27K 3/02, B27K 3/34, B27K 5/00

(54) **PURIFICATION OF WOOD ACETYLATION FLUID**
REINIGUNG EINER ACETYLIERUNGSFLÜSSIGKEIT AUS HOLZ
PURIFICATION DE FLUIDE D'ACÉTYLATION DU BOIS

(30) Priority: 28.07.2020 EP 20188106
(43) Date of publication of application: 07.06.2023
(73) Proprietor: Titan Wood Limited, London EC2R 6EA (GB)
(72) Inventor: RATERING, Peter Martinus Franciscus, London EC2R 6EA (GB); KAPPEN, Theodorus Gerardus Marinus Maria, London EC2R 6EA (GB)
(74) Representative: V.O.
(86) International application number: PCT/EP2021/071221
(87) International publication number: WO 2022/023452

(56) References cited:
- WO-A1-2016/009060
- CN-A- 106 800 605
- US-A- 6 026 656
- US-B2- 10 017 449
- US-B2- 7 612 232

## Description

### Field of the Invention

The invention relates to the purification of acetic acid recovered from a wood acetylation process, such as resulting in the removal of by-products and/or wood-based impurities. In particular, the invention relates to the removal of terpene and terpenoid impurities from the acid.

### Background

Acetylation of lignocellulosic materials, hereinafter referred to as wood, is a well-recognized process by which various properties of these materials can be improved. This relates, *inter alia,* to durability and dimensional stability.

Wood acetylation processes generally have in common that wood is subjected to contact with an acetylation fluid, under suitable acetylation conditions. A background reference is WO 2009/095687. Herein a process is described for the acetylation of wood, comprising the steps of submerging wood into an acetylation liquid in a reaction pressure vessel, conducting an impregnation procedure, removing excess acetylation fluid, introducing an inert fluid (typically nitrogen gas, the inert fluid possibly comprising noninert acetic anhydride and/or acetic acid) into the vessel, circulating and heating the inert fluid following a heating regime so as to bring about suitable acetylation of the wood, and removing the circulating fluid and allowing the acetylated wood to cool.

Acetylation fluids generally are selected from acetic acid, acetic anhydride, and mixtures thereof. After the wood acetylation process, utilized acetylation fluid is removed from the wood. It is thereby desired to avoid wasting the removed acetylation medium, e.g., by recirculating it, and re-using it in wood acetylation.

In many instances, the utilized acetylation fluid will comprise an excess of acetic acid, as this is formed as a by-product of the acetylation. It is desired to separate off such acetic acid, and put this to separate use, sell it as a chemical, and/or use it in the production of ketene.

However, the specific source of such acetic acid, viz. from the acetylation of wood, comes with inherent limitations to their further use due to the presence of terpene and terpenoid impurities from the wood. Other impurities that are desired to remove typically include by-products, such as acetonitrile and acetic acid esters such as methyl acetate and ethyl acetate. Wood acetylation may also result in the presence of chlorides in the utilized acetylation fluid. The chloride concentration in utilized acetylation fluid depends on the chloride content, and in general, of the chemical composition of the wood before acetylation. The level of chloride impurities present in wood may also depend on the wood species and on the region where the wood originated from; e.g., chlorides are particularly pronounced in rubberwood, more particularly if grown in coastal regions.

Terpenes and terpenoids include both native wood impurities, and conversion products that result from the heat to which the native wood terpenes and terpenoids are subjected during wood acetylation.

Such impurities, and particularly terpenes and terpenoids, are notoriously difficult to remove. This is not desirable when recirculating the acetylation fluid, particularly since obtaining desired high acetylation degrees in a controlled manner, requires the use of acetylation fluid of controlled purity. Moreover, the presence of impurities, particularly of terpene and terpenoid impurities, limits the further use, let alone separate sales, of acetic acid as recovered from wood acetylation. E.g., using it in a ketene furnace is not desired, as the aforementioned terpene and terpenoid impurities are prone to result in coke formation in the furnace, as a result of the high temperatures applied therein.

The purification of acetic acid recovered from wood acetylation is addressed in WO 2009/120257. Herein acetic acid comprising the aforementioned impurities is supplied to a distillation column together with water. The necessary addition of the amounts of water generally needed for such distillation presents a drawback in producing more diluted acetic acid than desired. In other respects too, such as heat energy consumption, the addition of water to the distillation column reduces the economic feasibility of the process. It is therefore desired to remove terpenes and terpenoids without distillation with water.

### Summary of the Invention

The invention, in one aspect, presents a process for purifying utilized acetylation fluid recovered from a process of acetylating wood, said recovered acetylation fluid comprising acetic acid, the process comprising subjecting the recovered acetylation fluid to cooling to below the melting point of acetic acid under the formation of crystals, and separating the crystals from the fluid.

In another aspect, the invention provides a process for removing one or more impurities selected from the group consisting of chlorides, acetonitrile, acetic acid esters such as methyl acetate and ethyl acetate, terpenes, terpenoids, and combinations thereof, from utilized acetylation fluid recovered from a process of acetylating wood, said recovered acetylation fluid comprising acetic acid, comprising subjecting the recovered acetylation fluid to cooling to below the melting point of acetic acid under the formation of crystals, and separating the crystals from the fluid.

The invention specifically includes, in a still further aspect, a process for removing one or more impurities selected from the group consisting of terpenes, terpenoids, and combinations thereof, from utilized acetylation fluid recovered from a process of acetylating a lignocellulosic material, said recovered acetylation fluid comprising acetic acid, comprising subjecting the recovered acetylation fluid to cooling to below the melting point of acetic acid under the formation of crystals, and separating the crystals from the fluid.

In yet another aspect, the invention presents a process for the acetylation of wood, comprising subjecting the wood to contact with an acetylation fluid comprising acetic anhydride and/or acetic acid under wood acetylation conditions, resulting in acetylated wood and utilized acetylation fluid comprising acetic acid, and purifying the utilized acetylation fluid by a process as described in any of the preceding paragraphs in this summary.

In a further aspect, the invention provides the use of acetic acid obtained by the purification method presented herein, as a reactant in the production of ketene from acetic acid, and/or in the production of acetic anhydride from acetic acid and ketene, as well as an integrated process for the acetylation of wood and the production of acetic anhydride.

### Detailed description

The invention puts to use the judicious insight to subject utilized acetylation fluid to cooling crystallization. It has thereby been found that acetic acid can be obtained which has been purified from terpenes of melting points not only below but also above that of acetic acid. This finding is surprising, as such terpenes would be expected to crystallize as well, or cocrystallize with acetic acid. Also, considering the notorious difficulty to remove terpene and terpenoid impurities from utilized acetylation fluid, it was unexpected that still another method could be found different from azeotropic distillation.

As an unexpected further finding, it is found to apply the cooling crystallization of utilized acetylation fluid in order to remove one or more impurities selected from the group consisting of chlorides, acetonitrile, water, acetic acid esters such as methyl acetate and ethyl acetate. Chlorides are typically ionic chlorides present in acetic acid after acetic acid / acetic anhydride separation followed by quenching with water. Without wishing to be bound by theory, the inventors believe that detected chlorides comprise hydrogen chloride and (generally hydrolysed) acetyl chloride. Other chlorides in the process may include stable organic chlorides and nonvolatile chlorides that remain with anhydride at anhydride/acetic acid separation.

The invention preferably pertains to the removal from utilized acetylation fluid of impurities that are terpenes, terpenoids, or a combination thereof. Terpenes are a large and diverse class of organic compounds, produced by a variety of plants, including trees. Terpenes are hydrocarbons. Terpenoids are modified terpenes, containing additional functional groups, usually oxygen-containing. Terpenes exist as monoterpenes as well oligoterpenes.

Terpenes may be classified by the number of isoprene units in the molecule; a prefix in the name indicates the number of terpene units needed to assemble the molecule. Hemiterpenes consist of a single isoprene unit. Isoprene itself is considered the only hemiterpene, but oxygen-containing derivatives such as prenol and isovaleric acid are hemiterpenoids. Monoterpenes consist of two isoprene units and have the molecular formula C₁₀H₁₆. Examples of monoterpenes and monoterpenoids include geraniol, terpineol, limonene, myrcene, linalool or pinene. Iridoids derive from monoterpenes. Sesquiterpenes consist of three isoprene units and have the molecular formula C₁₅H₂₄. Examples of sesquiterpenes and sesquiterpenoids include humulene, farnesenes, farnesol. Diterpenes are composed of four isoprene units and have the molecular formula C₂₀H₃₂. Examples of diterpenes and diterpenoids are cafestol, kahweol, cembrene and taxadiene. Sesterterpenes, terpenes having 25 carbons and five isoprene units, are rare relative to the other sizes. An example of a sesterterpenoid is geranylfarnesol. Triterpenes consist of six isoprene units and have the molecular formula C₃₀H₄₈. Sesquarterpenes are composed of seven isoprene units and have the molecular formula C₃₅H₅₆. Examples of sesquarterpenoids are ferrugicadiol and tetraprenylcurcumene. Tetraterpenes contain eight isoprene units and have the molecular formula C₄₀H₆₄.

Preferably, the process of the invention purifies the acetylation fluid from the main wood-based terpenes. Such wood-based terpenes comprise, *inter alia,* camphene, α-terpinolene, myrcene, α-pinene, β-pinene, p-cymene, 3-carene, and limonene.

The process of the invention is carried out with utilized acetylation fluid recovered from any wood acetylation process. Such wood acetylation processes include liquid phase processes, gas phase processes, and combinations thereof. Generally, wood to be acetylated will be impregnated with acetylation fluid, and subjected to one or more heating steps, generally under elevated pressure. The acetylation fluid can be acetic acid, acetic anhydride, or combinations thereof. Generally, the utilized acetylation fluid will comprise by-products from wood acetylation, extracted components from wood, such as terpenes and/or terpenoids, and excess acetylation fluid. Typically, when including acetic anhydride in the acetylation fluid, a by-product is acetic acid. Excess acetylation fluid will generally be acetic anhydride, acetic acid, or both.

Frequently, in the event of acetylation processes yielding a combination of acetic anhydride and acetic acid as a utilized acetylation fluid, it is desired to first separate the acetic acid from the acetic anhydride. This is generally done by distillation. As noted in the art, such distillation will result in acetic acid from which wood-derived impurities such as terpenes and/or terpenoids are not, or at least not sufficiently, removed.

In an embodiment, acetic anhydride is present in addition to the acetic acid in the utilized acetylation fluid that is subjected to cooling crystallization.

In an embodiment, the utilized acetylation fluid has been subjected to a step of removing acetic anhydride prior to being subjected to the cooling crystallization method of the present invention. It will be understood that the removal of acetic anhydride suitably is accomplished by distillation.

The process of the invention is generally conducted in accordance with optimized acetylation processes as are known in the field. Preferred processes comprise the following steps:
- Providing wood (solid wood or wood elements);
- Controlling, and if necessary adjusting, the moisture content of the wood or wood elements;
- Impregnating the wood or wood elements with acetylation fluid;
- Subjecting the impregnated wood or wood elements to one or more heating steps in order to effectuate acetylation of the wood elements;
- Separating the acetylated wood or wood elements from excess acetylation fluid.

Accordingly, the invention also relates to a process for the acetylation of wood, comprising subjecting the wood to contact with an acetylation fluid comprising acetic anhydride and/or acetic acid under wood acetylation conditions, resulting in acetylated wood and utilized acetylation fluid comprising acetic acid. In this process, the utilized acetylation fluid is subjected to purification by a method of cooling crystallization in accordance with any one or more of the embodiments described hereinbefore and hereinafter.

The acetylation process itself can be conducted, as known in the art, using liquid and/or gaseous acetylation fluid. Typical acetylation fluids are acetic acid, acetic anhydride, and mixtures thereof. Preferably the initial acetylation fluid used is acetic anhydride (as a result of the acetylation reaction, the composition of the acetylation fluid will change during the process, since acetic acid is thereby formed).

In interesting embodiments, the acetylation is conducted in accordance with any one of the acetylation processes as described in WO2009/095687, WO2011/95824, WO2013/117641, WO2013/139937, or WO2016/008995.

Acetylation reactions are generally conducted at temperatures of from 120°C to 200°C, such as 160°C to 180°C. The duration of the acetylation treatment generally ranges from 30 minutes to 3 hours. The skilled person will be able, for a given reactor equipment and depending on the wood species to be acetylated, to optimize the time and temperature conditions.

In the process of the invention, the utilized acetylation fluid is subjected to cooling crystallization. To this end, the recovered fluid is cooled to below the melting point of the utilized acetylation fluid. In embodiments in which the acetylation fluid has been subjected to substantial removal of acetic anhydride, the cooling will be below the melting point of acetic acid. Said melting point being 16.6°C, cooling will generally be to below this temperature, such as to a temperature in a range of from 0° to 16 °C, such as 10°C to 15°C. Lower temperatures can generally be applied, and will be applied in the event that the acetylation fluid has a lower freezing point, e.g. in the event of the presence of substantial amounts of lower melting compounds, such as water and/or acetic anhydride. The skilled person will be able to determine the freezing point of any fluid, such as utilized acetylation fluid, without difficulty.

In conducting the cooling crystallization method of the invention, it is preferred to recirculate the liquid that remains after crystallization, i.e., the mother liquor, rather than discard it after each round of crystallization. The recirculated fluid will thereby be replenished with fresh recovered acetylation fluid. This has the advantage of reducing waste, and it makes the process more economical. In preferred embodiments, such mother liquor is recirculated at least 20 times, such as at least 50 times, e.g., 20 to 200 times, preferably 50 to 100 times. Upon such recirculation, as a result of repeated crystallization of acetic acid, the concentration of possibly present liquids other than acetic acid will increase. E.g., if water is present in 0.1% in fresh recovered acetylation fluid, it will rise by-more than 5% after 60 times recycling in the recirculated mother liquor. In fact, water can be regarded as an impurity, which is present in relatively high content as compared to the other impurities, in the acetic acid, and will build up in the mother liquor (as will do all the other molecules that do not freeze). Mixing of such fresh recovered acetylation fluid with the recirculating mother liquor can occur batch-wise from a collecting vessel for utilized acetylation fluid. The crystallization process preferably is conducted as a continuous process. Thereby the fresh recovered utilized acetylation fluid can be still added in batches, or also as a continuous feed.

The additional liquids typically are acetic anhydride or water. The former will be present as a result of the original composition of the acetylation fluid, the amount dependent on the extent at which such anhydride is optionally removed before subjecting the utilized acetylation fluid to the crystallization method of the invention. Water can be present depending on the concentration of the acetic acid and/or acetic anhydride used as the wood acetylation fluid. Water can be added to quench the anhydride. This reduces the risk of corrosion in the crystallization equipment, as such quenching will effectively remove anhydride, thereby rendering the resulting process liquid (utilized acetylation liquid to be sent to crystallization) less corrosive. The process of the invention advantageously also works under such circumstances as having increased amounts of acetic anhydride and/or water present in the utilized acetylation fluid. This could not be predicted, in view of the lowering of the freezing point (or, put otherwise: the melting point) of acetic acid as a result of the presence of such additional liquids, notably in the event of water being present. E.g., the freezing point of acetic acid is lowered from 16°C to 5°C when 10% water is present.

The cooling can be conducted in any vessel or tube suitable for allowing crystals to be formed. The skilled person is familiar with suitable equipment, such as a scraped-wall crystalliser. It will be understood that, in order to carry out the present method, the crystallization equipment will allow cooling. Typically, crystals formed will be subjected to washing, generally in a wash column, e.g. to remove a liquid film of the mother liquor that typically remains in melt crystallization. The skilled person is wellknowledgeable on how to operate crystallization equipment.

In order to bring about crystallization, no special measures need to be taken. If desired, the process can be aided by adding a small amount, such as less than 10 wt.%, such as less than 5 wt.%, e.g. 1 wt.% to 3 wt.%, of a suitable contaminant, e.g. water or acetic anhydride, in order to aid initiating crystallization. The skilled person will be aware of techniques to facilitate the occurrence of crystallization, such as scratching the wall of the crystallizer, e.g., with a spatula.

It can be advantageous to boost crystallization by having one or more seed crystals of acetic acid present. Such seed crystals can be added to the fluid when the temperature thereof is about or below the melting point of acetic acid. Seed crystals will generally be obtained and stored in advance. Advantageously, they can also be obtained *in situ,* in the process of the invention.

In an interesting embodiment, the crystallization is conducted in two stages. Thereby, in a first step, the recovered acetylation fluid is subjected to cooling to below the melting point of acetic acid under the formation of crystals. This step can be conducted as above, with or without seed crystals. Subsequently, in a second step, a first portion of the crystals so obtained, generally after washing off any film of residual feed liquid, is subjected to melting so as to obtain an acetic acid melt. Since not all of the crystals are melted, a second portion thereof is retained. The acetic acid melt is recirculated and again subjected to cooling, to below the melting point of acetic acid. This cooling being done in the presence of at least part of the retained crystals, the second crystallization step is thus conducted in the presence of seeds. The recirculated melt can be subjected to cooling as such, or after being combined with a further amount of utilized acetylation fluid recovered from a wood acetylation process (either from the same process run, or from a different run thereof, or - e.g., if multiple acetylation reactors operate in parallel, from a different process). The aforementioned process can be repeated so as to provide multiple washing and recrystallization steps, generally leading to a further degree of purity.

In the two-staged embodiment, said first portion (i.e., the crystals to re-melted) comprises generally more than 50% of the crystals formed in the first step Preferably said first portion comprises 60% to 99% of the crystals formed in the first step, and more preferably 85% to 95% thereof. If desired, any third and subsequent stages can be conducted: after the crystallization of the second step, a portion of the crystals then formed can be re-melted again, and the process continued as above. Thus, wood acetylation and recovery of acetylation fluid with the purification process of the invention, can be an ongoing continuous or semi-continuous operation.

In another interesting embodiment, seed crystals are separately added, such as provided from storage, not requiring re-melting crystals obtained from the same acetylation fluid being crystallized. An advantage of this embodiment is that it more easily facilitates conducting the crystallization in a continuous process. It will be understood that also the continuous process may advantageously involve re-melting and recrystallization.

A combination of the foregoing embodiments is also conceivable. Hereby, in any stage, seed crystals can be chosen to be from storage, or from *in situ* crystal formation. E.g., in a first stage seed crystals can be provided from storage, and one or more subsequent stages, seed crystals are obtained by leaving part of the formed crystals out of re-melting.

The wood to be acetylated is either in the form of wood elements or solid wood, and includes also wood veneers. The wood elements can preferably be, e.g., wood chips, wood strands, wood particles. The wood preferably belong to non-durable wood species such as soft woods, for example, coniferous trees, typically spruce, pine or fir, or to non-durable hardwoods, such as rubberwood. Non-limiting examples of suitable types of wood are spruce, sitka spruce, maritime pine, scots pine, radiata pine, eucalyptus, red alder, European alder, beech, birch. loblolly pine, lodgepole pine, pitch pine, red pine, Southern yellow pine, Japanese cedar (sugi), and hemlock. Also suitable are monocots, such as palm, and other hardwoods, such as Paulownia, rubberwood, teak, maple, oak, white oak, and the like.

Typically, the wood to be acetylated is not wood pulp. Particularly, wood acetylation processes are distinguished from processes in which wood-based starting materials such as pulp are subjected to chemical reactions involving the formation of new materials and/or shapes, such as in making nanocellulose from cellulose-based starting materials. Essentially, wood acetylation processes serve to retain the wood (solid wood, wood veneers, wood elements), in its original shape, and only change the wood to the extent that it becomes acetylated. Particularly, other than in the case of e.g. nanocellulose, acetylated wood contains, besides cellulose, also hemicellulose and, notably, lignin. The effect of acetylating the wood is to acetylate these wood components, resulting in the presence of acetylated cellulose, acetylated hemicellulose and acetylated lignin.

Typical dimensions of wood elements subjected to acetylation are given in the following table.

**Table 1**

| wood element | length (mm) | | width (mm) | | thickness (mm) | |
|---|---|---|---|---|---|---|
| | from | to | from | to | from | to |
| Chips | 5 | 75 | 5 | 50 | 1,5 | 25 |
| Strands | 20 | 120 | 5 | 40 | 0,25 | 1,5 |
| splinters (slivers) | 5 | 75 | 0,15 | 0,5 | 0,15 | 0,5 |
| Particles | 1,5 | 20 | 0,15 | 5 | 0,15 | 5 |
| Fibre bundles | 1,5 | 25 | 0,15 | 0,5 | 0,15 | 0,5 |
| Fibres | 1 | 5 | 0,05 | 0,1 | 0,05 | 0,1 |

In some embodiments, the wood elements have a length 1.0-75 mm, a width of 0.05-75mm and a thickness of 0.05-15 mm.

In alternative embodiments, the wood is solid wood or veneers of wood and preferably has a length or width of at least 8 cm. The thickness preferably is at least 1mm. In some embodiments, the wood has a width of 2 cm to 30 cm, a thickness of 2 cm to 16 cm and a length of from 1.5 to 6.0 m. In other embodiments, the wood has a thickness of at least 1mm, a width of 20 cm - 2.5 m and length of 20cm to 6m.

In sum, the present disclosure provides a method of purification of utilized acetylation fluid recovered from a process of acetylating wood. The method involves subjecting the recovered acetylation fluid to cooling crystallization. By this method terpene and terpenoid impurities are removed, as well as other impurities. Disclosed is also a method of acetylating wood resulting in acetylated wood and utilized acetylation fluid comprising acetic acid, and purifying the utilized acetylation fluid by cooling crystallization.

It will be understood that the product obtained from the purification method described hereinbefore, is purified acetic acid. This resulting purified acetic acid can be put to separate use, be sold as a chemical, and particularly can be used in the production of acetic anhydride, ketene or more generally acetylating agents, acetylating agents building blocks, intermediates or components of acetylation fluids.

The skilled person is fully familiar with methods of producing ketene from acetic acid, as well as with producing acetic anhydride from acetic acid and ketene. The ketene manufacturing process generally comprises dehydrating acetic acid by subjecting it to heat. This typically occurs in a ketene furnace, operated at, temperatures of the order of, e.g., 700°C to 750°C. Acetic anhydride can be formed in an exothermic reaction by reacting ketene with acetic acid. The present invention includes the use of acetic acid obtained by the purification method presented in this disclosure, in all its embodiments, as a reactant in the production of ketene from acetic acid, as a reactant in the production of acetic anhydride from acetic acid and ketene, or as a reactant in both. The ketene used in the production of the acetic anhydride can be sourced from elsewhere, but preferably is obtained in a ketene-manufacturing process coupled to the acetic anhydride production process. Either or both of the ketene manufacturing process and the acetic anhydride process, are coupled to a wood acetylation process from which acetylation fluid is recovered and purified according to the crystallization method described herein. This coupling is such that the purified acetic acid obtained, is led to either of both of the ketene manufacturing process and the acetic anhydride manufacturing process. This can be accomplished, e.g., in accordance with the general process scheme of Fig. 1 in WO 2016/09060. This involves sending acetic acid, which is obtained after separating this from acetic anhydride ("ACA/ANH Separation") in recovered utilized acetylation fluid from wood acetylation, to either or both of a ketene furnace ("Cracking Furnace") and a reactor for the production of acetic anhydride ("ANH Reaction"). In this embodiment of the present invention, a cooling crystallization process as described hereinbefore, is implemented downstream of the ACA/ANH separation, and upstream of both the cracking furnace and the ANH Reaction. Preferably, this enables an integrated process for the acetylation of wood and the production of acetic anhydride. Therein the process for the acetylation of wood comprises subjecting the wood to contact with an acetylation fluid comprising acetic anhydride and/or acetic acid under wood acetylation conditions. This results in acetylated wood and utilized acetylation fluid comprising acetic acid, whereby the process comprises purifying the utilized acetylation fluid by cooling crystallization as substantially described hereinbefore, in all embodiments. The crystallization process of the invention results in obtaining purified acetic acid, wherein the purified acetic acid, optionally mixed with fresh acetic acid, is used in the production of ketene and acetic anhydride as described above. The produced anhydride can then be further used as, or as a component of, the acetylation fluid in the aforementioned process for the acetylation of wood.

It will be understood that variations of coupled processes are well possible. E.g., the purified acetic acid can be used in the production of ketene, whilst the production of acetic anhydride employs fresh acetic acid, or the other way around. Or, e.g., the produced acetic anhydride is not used in the acetylation of wood, but put to use or sales outside of the integrated process. These and other variations will be clear to the skilled person.

Other uses for the resulting purified acetic acid are for the production of vinyl acetate monomer to be polymerized to poly(vinyl acetate) and other polymers, for the production of esters to be used as solvents in inks, paints, and coatings, and in food applications.

The invention is illustrated with reference to the following, non-limiting examples.

### Examples

Unless otherwise stated, all part-per-million (ppm), percentage (%) and ratio values correspond to milligram per kilogram, weight percent and weight ratio values respectively. The density of all solutions mentioned in these examples is considered equal.

Unless otherwise stated, the experiments disclosed in the present document were performed with impure acid produced by distillation of the utilized acetylation fluid of the industrial wood acetylation plant producing Accoya^{®} wood. This impure acetic acid contained about 1% acetic anhydride, several terpenes in concentrations ranging from less than 10 to more than hundred ppm, about a thousand ppm methyl acetate and about 50 ppm of ethyl acetate and acetonitrile. Impurities are to be understood as terpenes, terpenoids, acetic acid esters, acetonitrile, or a combination thereof.

Impure acetic acid is to be understood as a solution containing acetic acid as the main component and impurities to be removed by the present invention.

Unless otherwise stated, when water was added to impure acetic acid containing acetic anhydride, the mixture was kept for a sufficient amount of time for the hydrolysis of acetic anhydride to be completed.

Impure acetic acid, purified acetic acid and mother liquor were analysed by Gas Chromatography, in order to determine the concentration in organic impurities. Quenched impure acetic acid, quenched purified acetic acid and quenched mother liquor can be analysed by argentometric titration, in order to determine the concentration in chloride. The results are given in ppm and in % of the concentration in the original impure acetic acid. The experimental and analytical accuracy was evaluated by mass balance control on each impurity.

Unless otherwise stated, the experimental crystallization setup consisted of a tubular crystallizer with a jacket equipped with a circulation cooler. Through the inner part of the tube the acetic acid was continuously recirculated using a peristaltic pump. Before the start of crystallization, the residence time in the crystallizer was measured to be 1.5 minutes. Cooling water was pumped through the jacket of the tube at such a flow rate that the temperature in the cooling jacket was uniform at every point. Temperature equilibrium was considered to be reached when the temperature of the acetic acid getting out of the crystallization set-up was not changing by more than 1 degree Celsius per minute.

Unless otherwise stated, the crystals produced were washed with a minimal amount of glacial acetic acid at room temperature before being melted.

The following impurities are referred to in the Examples:
3C = 3-carene;
αP = alpha-pinene;
βP = beta-pinene;
αTA = alpha-terpinyl acetate;
fen = fenchone;
lim = limonene;
cym = p-cymene;
cam = camphene;
ACN = acetonitrile;
EAc = ethyl acetate;
MAc = methyl acetate;
αT = alpha-terpinene.

### Example 1

An experiment was performed with acetic acid obtained by distillation from utilized acetylation fluid obtained from an industrial wood acetylation plant producing Accoya^{®} acetylated solid wood.

Prior to the experiment, a sample of the acetic acid was analysed in a Gas Chromatography column, in order to determine the amount of impurities.

The experimental setup consisted of a glass tube with a jacket. Through the inner part of the tube the acetic acid was continuously recirculated. Cold (14°C) water was pumped through the jacket of the tube after which it was disposed to the drain. Two rotary pumps were used, one to recirculate acetic acid through the closed loop, and another one to pump the cold water through the jacket.

In order to bring about crystallization, after 30-40 minutes of cooling, the acetic acid loop was stopped and the bottom of the tube was closed. A thermometer was gently inserted at the top of the tube, causing motion and increasing the surface in the system. This resulted in an almost instantaneous crystallization of acetic acid throughout the tube.

Next, the acetic acid was melted for about 90% (by visual determination), leaving about 10% of the crystals in order to serve as a seed for subsequent crystallization. After said melting, the acetic acid loop was started again. This time crystallization was significantly slower and acetic acid crystals covered most of the walls and inner part of the tube.

On average the impurities (mainly terpenes) were removed to an extent of 40% to 50%. The removal was consistent for all terpene impurities determined, irrespective of, e.g., the melting point of the terpenes. It is unexpected that terpenes with a higher freezing point than that of acetic acid are removed, such as camphene (freezing point 50°C-51°C). This also holds for the embodiments in which, as a result of water and/or anhydride being present, the melting point of acetic acid is in fact lowered. E.g., the terpene fenchone (melting point 6°C) is effectively removed from acetic acid both in the absence and in the presence of water.

The removal of impurities is considerably improved by washing the crystals with purified acetic acid. Further increased removal rates are obtained with re-melting and recrystallizing the washed acetic acid crystals.

### Example 2

100 mL of impure acetic acid labelled Ba was circulated in the crystallization set-up. The cooling water was set to 13.0°C. The acid and the cold water were circulated until temperature equilibrium was reached. Then, 50 mg of crystalline acetic acid seeds were placed in the crystallization zone of the set-up, that triggered crystallization of the impure acetic acid. After 15 minutes, the mother liquor labelled Bb was drained from the set-up. 77 mL of mother liquor was retrieved. The cooling of the set-up was stopped, and the crystals were melted, resulting in 18 mL of purified acetic acid labelled Bd.

The analytical results showed an enrichment of the mother liquor and a depletion of the purified acetic acid in all impurities. The concentrations in ppm and concentrations relative to the initial impure acetic acid are displayed in tables 2 and 3 respectively.

**Table 2**

| Concentration in terpenes of the impure acetic acid Ba, of the mother liquor Bb and of the purified acetic acid Bd (in ppm) | | | | | | | | | |
|---|---|---|---|---|---|---|---|---|---|
| | **3C** | **αP** | **αTA** | **fen** | **lim** | **cym** | **cam** | **ACN** | **EAc** |
| Ba | 93 | 64 | 7 | 19 | 190 | 29 | 47 | 49 | 68 |
| Bb | 109 | 83 | 8 | 25 | 227 | 35 | 55 | 55 | 79 |
| Bd | 21 | 16 | 3 | 4 | 40 | 8 | 12 | 17 | 16 |

**Table 3**

| Concentration in terpenes of the mother liquor Bb and of the purified acid Bd relative to the impure acetic acid Ba (in %) | | | | | | | | | |
|---|---|---|---|---|---|---|---|---|---|
| | **3C** | **αP** | **αTA** | **fen** | **lim** | **cym** | **cam** | **ACN** | **EAc** |
| Bb | 118% | 130% | 117% | 130% | 119% | 118% | 117% | 112% | 117% |
| Bd | 22% | 24% | 51% | 19% | 21% | 28% | 25% | 35% | 24% |

### Example 3

In this example the present purification of acetic acid is conducted in the presence of relatively large amounts of impurities (such as will occur, e.g., upon repeated recirculation). To this end, 80 mL of impure acetic acid was further contaminated by technical grade limonene, 3-carene, alpha-pinene, camphene, para-cymene, beta-pinene and methyl acetate. The highly impure acetic acid produced was labelled Ca.

Ca was circulated in the crystallization set-up. The cooling water was set to 14.0°C. The acid and the cold water were circulated until temperature equilibrium was reached. Then, 50 mg of crystalline acetic acid seeds were placed in the crystallization zone of the set-up, that triggered crystallization of the highly impure acetic acid. After 1h, the mother liquor labelled Cb was drained from the set-up. 73 mL of mother liquor was retrieved. The cooling of the set-up was stopped, and the crystals were melted, resulting in 10 mL of purified acetic acid labelled Cd.

The analytical results show an enrichment of the mother liquor in almost all impurities and a depletion of the purified acetic acid in all impurities. The concentrations in ppm and concentrations relative to the initial impure acetic acid are displayed in tables 4 and 5 respectively.

**Table 4**

| Concentration in terpenes of the highly impure acetic acid Ca, of the mother liquor Cb and of the purified acetic acid Cd (in ppm) | | | | | | | | | | | | |
|---|---|---|---|---|---|---|---|---|---|---|---|---|
| | **3C** | **αP** | **αTA** | **fen** | **lim** | **cym** | **cam** | **αT** | **βP** | **ACN** | **EAc** | **MAc** |
| **Ca** | 2439 | 1935 | 12 | 24 | 2137 | 1907 | 1347 | 11 | 2443 | 479 | 1626 | 13156 |
| **Cb** | 2619 | 2106 | 23 | 24 | 2309 | 2095 | 1480 | 11 | 2605 | 479 | 1685 | 12553 |
| **Cd** | 323 | 248 | 5 | 5 | 285 | 231 | 168 | 3 | 299 | 64 | 142 | 964 |

**Table 5**

| Concentration in impurities of the mother liquor Cb and of the purified acid Cd relative to the impure acetic acid Ca (in %) | | | | | | | | | | | | |
|---|---|---|---|---|---|---|---|---|---|---|---|---|
| | **3C** | **αP** | **αTA** | **fen** | **lim** | **cym** | **cam** | **αT** | **βP** | **ACN** | **EAc** | **MAc** |
| **Cb** | 107 | 109 | 193 | 101 | 108 | 110 | 110 | 103 | 107 | 100 | 104 | 95 |
| **Cd** | 13 | 13 | 41 | 21 | 13 | 12 | 12 | 26 | 12 | 13 | 9 | 7 |

### Example 4

A mixture made of 95 mL of impure acetic acid and 5 mL of water labelled Da was circulated in the crystallization set-up. The cooling water was set to 7.0°C. The mixture Da and the cold water were circulated until temperature equilibrium was reached. Then, 50 mg of crystalline acetic acid seeds were placed in the crystallization zone of the set-up, that triggered crystallization of the impure acetic acid. The cooling water was set to 5.0°C. After 20 minutes, the mother liquor labelled Db was drained from the setup. 88 mL of mother liquor was retrieved. The cooling of the set-up was stopped, and the crystals were melted, resulting in 12 mL of purified acetic acid labelled Dd.

The analytical results show an enrichment of the mother liquor in most of the impurities and a depletion of the purified acetic acid in all impurities. The concentrations in ppm and concentrations relative to the initial impure acetic acid are displayed in tables 6 and 7 respectively.

**Table 6**

| Concentration in impurities of the acetic acid Da, of the mother liquor Db and of the purified acetic acid Dd (in ppm) | | | | | | | | | | |
|---|---|---|---|---|---|---|---|---|---|---|
| | **3C** | **αP** | **αTA** | **fen** | **lim** | **cym** | **cam** | **ACN** | **EAc** | **MAc** |
| **Da** | 89 | 41 | 14 | 26 | 180 | 29 | 26 | 50 | 66 | 416 |
| **Db** | 91 | 46 | 15 | 19 | 175 | 29 | 22 | 51 | 69 | 470 |
| **Dd** | 32 | 18 | 5 | 8 | 54 | 12 | 11 | 20 | 19 | 143 |

**Table 7**

| Concentration in impurities of the mother liquor Db and of the purified acid Dd relative to the impure acetic acid Da (in %) | | | | | | | | | | |
|---|---|---|---|---|---|---|---|---|---|---|
| | **3C** | **αP** | **αTA** | **fen** | **lim** | **cym** | **cam** | **ACN** | **EAc** | **MAc** |
| **Db** | 102 | 112 | 105 | 71 | 97 | 100 | 85 | 104 | 105 | 113 |
| **Dd** | 36 | 45 | 35 | 29 | 30 | 42 | 44 | 41 | 29 | 34 |

## Claims

1. A process for purifying utilized acetylation fluid recovered from a process of acetylating wood, said recovered acetylation fluid comprising acetic acid, the process comprising subjecting the recovered acetylation fluid to cooling to below the melting point of acetic acid under the formation of crystals, and separating the crystals from the fluid.

2. A process according to claim 1, comprising removing impurities selected from the group consisting of chlorides, acetonitrile, acetic acid esters such as methyl acetate and ethyl acetate, terpenes, terpenoids, and combinations thereof.

3. A process according to claim 1 or 2, wherein the wood is selected from the group consisting of solid wood, wood veneers, and wood elements.

4. A process according to any one of the preceding claims, wherein the process of acetylating wood comprises subjecting the wood to an acetylation fluid comprising acetic anhydride, preferably consisting of acetic anhydride.

5. A process according to any one of the preceding claims, comprising cooling the recovered acetylation fluid in the presence of one or more seed crystals of acetic acid.

6. A process according to claim 5, comprising the steps of (a) subjecting the recovered acetylation fluid to cooling to below the melting point of acetic acid under the formation of crystals; (b) subjecting a first portion of the crystals to melting so as to obtain an acetic acid melt, thereby retaining a second portion of the crystals; (c) recirculating said acetic acid melt; (d) subjecting said melt to cooling to below the melting point of acetic acid in the presence of at least part of the retained crystals.

7. A process according to claim 6, wherein said first portion comprises 60% to 99% of the crystals formed in step (a), preferably 85% to 95% thereof.

8. A process according to any one of the claims 5 to 7, comprising separately adding seed crystals.

9. A process according to any one of the preceding claims, wherein the recovered acetylation fluid comprises acetic anhydride and acetic acid.

10. A process according to any one of the preceding claims, comprising subjecting the recovered acetylation fluid to removal of acetic anhydride, such as by distillation, before said fluid is subjected to said cooling.

11. A process according to claim 10, wherein the cooling is to a temperature below the melting point of acetic acid.

12. A process according to claim 11, wherein the cooling is to a temperature in a range of from 0° to 16 °C, preferably 10°C to 15°C.

13. A process according to any one of the preceding claims, comprising subjecting the formed crystals to washing.

14. A process according to any one of the preceding claims, comprising a plurality of subsequent melting and re-crystallizing steps.

15. A process according to any one of the preceding claims, wherein the fluid remaining after separation of the crystals is recirculated and again subjected to the steps of cooling and crystallization, said recirculation being conducted 20 to 200 times, preferably 50 to 100 times.

16. A process for the acetylation of wood, comprising subjecting the wood to contact with an acetylation fluid comprising acetic anhydride and/or acetic acid under wood acetylation conditions, resulting in acetylated wood and utilized acetylation fluid comprising acetic acid, and purifying the utilized acetylation fluid by a process as defined in any of the preceding claims.

17. A process for the production of ketene comprising purifying utilized acetylation fluid by a process according to any one of the claims 1 to 15 so as to obtain acetic acid, and subjecting said acetic acid to dehydration so as to produce ketene.

18. A process for the production of acetic anhydride comprising purifying utilized acetylation fluid by a process according to any one of the claims 1 to 15 so as to obtain acetic acid, and subjecting said acetic acid to reaction with ketene so as to produce acetic anhydride.

19. The process of claim 17 or 18 wherein said process to produce ketene and said process to produce acetic anhydride are coupled such that the produced ketene is used as a reactant in the production of acetic anhydride, wherein the purified acetic acid, optionally mixed with acetic acid from another source, is used in the production of ketene, in the production of acetic anhydride, or in both.

20. An integrated process for the acetylation of wood and the production of acetic anhydride, wherein the process for the acetylation of wood is a process according to claim 16, thereby obtaining purified acetic acid, wherein the purified acetic acid, optionally mixed with fresh acetic acid, is used in the production of ketene and acetic anhydride as defined in claim 19, and wherein the acetic anhydride thereby obtained, is provided to the acetylation fluid used in said process for the acetylation of wood.

## Patentansprüche

1. Verfahren zur Reinigung von verwendeter
Acetylierungsflüssigkeit, rückgewonnen aus einem Verfahren zur Acetylierung von Holz, die rückgewonnene Acetylierungsflüssigkeit umfassend Essigsäure, das Verfahren umfassend Unterziehen der rückgewonnenen Acetylierungsflüssigkeit einer Abkühlung unter den Schmelzpunkt von Essigsäure unter Bildung von Kristallen, und Trennen der Kristalle von der Flüssigkeit.

2. Verfahren nach Anspruch 1, umfassend Entfernen von Verunreinigungen, ausgewählt aus der Gruppe bestehend aus Chloriden, Acetonitril, Essigsäureestern wie Methylacetat und Ethylacetat, Terpenen, Terpenoiden und Kombinationen davon.

3. Verfahren nach Anspruch 1 oder 2, wobei das Holz ausgewählt wird aus der Gruppe bestehend aus Massivholz, Holzfurnieren und Holzelementen.

4. Verfahren nach einem der vorhergehenden Ansprüche, wobei das Verfahren zum Acetylieren von Holz Unterziehen des Holzes einer Acetylierungsflüssigkeit, umfassend Essiganhydrid, umfasst, vorzugsweise bestehend aus Essiganhydrid.

5. Verfahren nach einem der vorhergehenden Ansprüche, umfassend Abkühlen der rückgewonnenen Acetylierungsflüssigkeit in Gegenwart eines oder mehrerer Essigsäure-Impfkristalle.

6. Verfahren nach Anspruch 5, umfassend die Schritte (a)Unterziehen der rückgewonnenen Acetylierungsflüssigkeit dem Abkühlen unter den Schmelzpunkt von Essigsäure unter Bildung von Kristallen; (b) Unterziehen eines ersten Teils der Kristalle dem Schmelzen, um so eine Essigsäureschmelze zu erhalten, wodurch ein zweiter Teil der Kristalle zurückgehalten wird; (c) Umwälzens der Essigsäureschmelze; (d) Unterziehen der Schmelze der Abkühlung unter den Schmelzpunkt von Essigsäure in Anwesenheit mindestens eines Teils der zurückgehaltenen Kristalle.

7. Verfahren nach Anspruch 6, wobei der erste Teil 60 % bis 99 % der in Schritt (a) gebildeten Kristalle, vorzugsweise 85 % bis 95 % davon, umfasst.

8. Verfahren nach einem der Ansprüche 5 bis 7, umfassend das getrennte Hinzufügen von Impfkristallen.

9. Verfahren nach einem der vorhergehenden Ansprüche, wobei die rückgewonnene Acetylierungsflüssigkeit Essiganhydrid und Essigsäure umfasst.

10. Verfahren nach einem der vorhergehenden Ansprüche, umfassend Unterziehen rückgewonnenen Acetylierungsflüssigkeit Entfernen von Essiganhydrid, wie durch Destillation, bevor die Flüssigkeit der Abkühlung unterzogen wird.

11. Verfahren nach Anspruch 10, wobei die Kühlung auf eine Temperatur unterhalb des Schmelzpunkts von Essigsäure erfolgt.

12. Verfahren nach Anspruch 11, wobei die Abkühlung auf eine Temperatur in einem Bereich von 0 °C bis 16 °C, vorzugsweise 10 °C bis 15 °C, ist.

13. Verfahren nach einem der vorhergehenden Ansprüche, umfassend Unterziehen der gebildeten Kristalle dem Waschen.

14. Verfahren nach einem der vorhergehenden Ansprüche, umfassend eine Vielzahl an nachfolgenden Schmelz- und Rekristallisierungsschritten.

15. Verfahren nach einem der vorhergehenden Ansprüche, wobei die nach der Trennung der Kristalle verbliebene Flüssigkeit umgewälzt wird und erneut den Schritten des Abkühlens und der Kristallisierung unterzogen wird, wobei die Umwälzung 20- bis 200-mal, vorzugsweise 50- bis 100-mal, durchgeführt wird.

16. Verfahren zur Acetylierung von Holz, umfassend Unterziehen des Holzes dem in Kontakt bringen mit Acetylierungsflüssigkeit, umfassend Essiganhydrid und/oder Essigsäure, unter Holzacetylierungsbedingungen, resultierend in acetyliertem Holz und verwendeter Acetylierungsflüssigkeit, umfassend Essigsäure, und Reinigen der verwendeten Acetylierungsflüssigkeit durch ein Verfahren gemäß einem der vorhergehenden Ansprüche.

17. Verfahren zur Herstellung von Keten, umfassend Reinigen der verwendeten Acetylierungsflüssigkeit durch ein Verfahren nach einem der Ansprüche 1 bis 15, um so Essigsäure zu erhalten, und Unterziehen der Essigsäure dem Dehydrieren, um Keten herzustellen.

18. Verfahren zur Herstellung von Essiganhydrid, umfassend Reinigen verwendeter Acetylierungsflüssigkeit durch ein Verfahren nach einem der Ansprüche 1 bis 15, um so Essigsäure zu erhalten, und Unterziehen der Essigsäure einer Reaktion mit Keten, um Essiganhydrid herzustellen.

19. Verfahren nach Anspruch 17 oder 18, wobei das Verfahren zur Herstellung von Keten und das Verfahren zur Herstellung von Essiganhydrid gekoppelt sind, dass das hergestellte Keten als Reaktant bei der Herstellung von Essiganhydrid verwendet wird, wobei die gereinigte Essigsäure, optional gemischt mit Essigsäure aus einer anderen Quelle, bei der Herstellung von Keten, bei der Herstellung von Essiganhydrid oder beiden verwendet wird.

20. Integriertes Verfahren zur Acetylierung von Holz und zur Herstellung von Essiganhydrid, wobei das Verfahren zur Acetylierung von Holz ein Verfahren nach Anspruch 16 ist, wodurch gereinigte Essigsäure erhalten wird, wobei die gereinigte Essigsäure, optional gemischt mit frischer Essigsäure, bei der Herstellung von Keten und Essiganhydrid wie in Anspruch 19 definiert verwendet wird und wobei das dadurch erhaltene Essiganhydrid der in dem genannten Verfahren zur Acetylierung von Holz verwendeten Acetylierungsflüssigkeit bereitgestellt wird.

## Revendications

1. Procédé de purification d'un fluide d'acétylation utilisé récupéré à partir d'un procédé d'acétylation du bois, ledit fluide d'acétylation récupéré comprenant de l'acide acétique, le procédé comprenant l'exposition du fluide d'acétylation récupéré à un refroidissement jusqu'en dessous du point de fusion de l'acide acétique sous la formation de cristaux, et la séparation des cristaux et du fluide.

2. Procédé selon la revendication 1, comprenant l'élimination d'impuretés sélectionnées dans le groupe consistant en des chlorures, de l'acétonitrile, des acide acétique esters tels que méthyle acétate et éthyle acétate, des terpènes, des terpénoïdes, et des combinaisons de ceux-ci.

3. Procédé selon la revendication 1 ou 2, dans lequel le bois est sélectionné dans le groupe consistant en du bois massif, des placages de bois, et des éléments en bois.

4. Procédé selon l'une quelconque des revendications précédentes, dans lequel le procédé d'acétylation du bois comprend l'exposition du bois à un fluide d'acétylation comprenant de l'anhydride acétique, de préférence consistant en de l'anhydride acétique.

5. Procédé selon l'une quelconque des revendications précédentes, comprenant le refroidissement du fluide d'acétylation récupéré en présence d'un ou plusieurs cristaux germes d'acide acétique.

6. Procédé selon la revendication 5, comprenant les étapes choisies parmi (a) l'exposition du fluide d'acétylation récupéré à un refroidissement jusqu'en dessous du point de fusion de l'acide acétique sous la formation de cristaux ; (b) l'exposition d'une première partie des cristaux à une fusion de manière à obtenir une masse fondue d'acide acétique, en conservant ainsi une seconde partie des cristaux ; (c) la remise en circulation de ladite masse fondue d'acide acétique ; (d) l'exposition de ladite masse fondue à un refroidissement jusqu'en dessous du point de fusion de l'acide acétique en présence d'au moins une partie des cristaux conservés.

7. Procédé selon la revendication 6, dans lequel ladite première partie comprend 60 % à 99 % des cristaux formés à l'étape (a), de préférence 85 % à 95 % de ceux-ci .

8. Procédé selon l'une quelconque des revendications 5 à 7, comprenant l'ajout séparé de cristaux germes.

9. Procédé selon l'une quelconque des revendications précédentes, dans lequel le fluide d'acétylation récupéré comprend de l'anhydride acétique et de l'acide acétique.

10. Procédé selon l'une quelconque des revendications précédentes, comprenant l'exposition du fluide d'acétylation récupéré à une élimination d'anhydride acétique, telle que par distillation, avant que ledit fluide ne soit soumis audit refroidissement.

11. Procédé selon la revendication 10, dans lequel le refroidissement s'effectue jusqu'à une température en dessous du point de fusion de l'acide acétique.

12. Procédé selon la revendication 11, dans lequel le refroidissement s'effectue jusqu'à une température dans une plage de 0 ° à 16 °C, de préférence de 10 °C à 15 °C.

13. Procédé selon l'une quelconque des revendications précédentes, comprenant l'exposition des cristaux formés à un lavage.

14. Procédé selon l'une quelconque des revendications précédentes, comprenant une pluralité d'étapes ultérieures de fusion et de recristallisation.

15. Procédé selon l'une quelconque des revendications précédentes, dans lequel le fluide restant après séparation des cristaux est remis en circulation et soumis à nouveau aux étapes de refroidissement et de cristallisation, ladite remise en circulation étant réalisée 20 à 200 fois, de préférence 50 à 100 fois.

16. Procédé d'acétylation du bois, comprenant la mise en contact du bois avec un fluide d'acétylation comprenant de l'anhydride acétique et/ou de l'acide acétique sous des conditions d'acétylation du bois, résultant en du bois acétylé et un fluide d'acétylation utilisé comprenant de l'acide acétique, et la purification du fluide d'acétylation utilisé par un procédé tel que défini dans l'une quelconque des revendications précédentes.

17. Procédé de production de cétène comprenant la purification de fluide d'acétylation utilisé par un procédé selon l'une quelconque des revendications 1 à 15 de manière à obtenir de l'acide acétique, et l'exposition dudit acide acétique à une déshydratation de manière à produire du cétène.

18. Procédé de production d'anhydride acétique comprenant la purification de fluide d'acétylation utilisé par un procédé selon l'une quelconque des revendications 1 à 15 de manière à obtenir de l'acide acétique, et l'exposition dudit acide acétique à une réaction avec du cétène de manière à produire de l'anhydride acétique.

19. Procédé selon la revendication 17 ou 18, dans lequel ledit procédé de production de cétène et ledit procédé de production d'anhydride acétique sont couplés de telle sorte que le cétène produit est utilisé comme réactif dans la production d'anhydride acétique, dans lequel l'acide acétique purifié, facultativement mélangé à de l'acide acétique provenant d'une autre source, est utilisé dans la production de cétène, dans la production d'anhydride acétique, ou dans les deux.

20. Procédé intégré d'acétylation du bois et de production d'anhydride acétique, dans lequel le procédé d'acétylation du bois est un procédé selon la revendication 16, obtenant ainsi de l'acide acétique purifié, dans lequel l'acide acétique purifié, facultativement mélangé à de l'acide acétique frais, est utilisé dans la production de cétène et d'anhydride acétique telle que définie dans la revendication 19, et dans lequel l'anhydride acétique ainsi obtenu est fourni au fluide d'acétylation utilisé dans ledit procédé d'acétylation du bois.
